# EUROPÄISCHE PATENTANMELDUNG

(11) **EP 1 352 669 A1**
(43) Veröffentlichungstag der Anmeldung: **15.10.2003**
(21) Anmeldenummer: 03007707.7
(22) Anmeldetag: 03.04.2003
(51) Int. Cl.: A61M 13/00

(54) **Verfahren zur Insufflation von Gas**

(30) Priorität: 11.04.2002 DE 10215955
(71) Anmelder: WISAP GESELLSCHAFT FÜR WISSENSCHAFTLICHEN APPARATEBAU MBH, D-82054 Sauerlach (DE)
(72) Erfinder: Pagel, Lienhard, Dr., 18311 Hirschburg (DE)
(74) Vertreter: Heim, Hans-Karl, Dipl.-Ing.

(57) **Zusammenfassung**

Die Erfindung betrifft ein Verfahren zur Insufflation von Gas mittels eines Insufflations-Systems in einen Körper, insbesondere in das Abdomen. Die Erfindung geht hierbei den Weg ein quasi-kontinuierliches Flow-Verfahren zu ermöglichen, wobei auch mit Überdruck insuffliert werden kann, aber Flow-Pausen vermieden werden. Der intraabdominelle Druck wird hierbei mit hinreichender Genauigkeit bestimmt.

## Beschreibung

Die Erfindung betrifft ein Verfahren zur Insufflation von Gas mittels eines Insufflations-Systems in einen Körper, insbesondere in das Abdomen.

Verfahren dieser Art werden in der minimal invasiven Chirurgie bereits eingesetzt.

Exemplarisch wird hierzu auf das Verfahren gemäß der DE 30 00 218 C3 hingewiesen, das als Verfahren nach dem Überdruckprinzip bezeichnet werden kann. Ein anderes Verfahren ist in der EP 169 972 B1 beschrieben, das eine Berechnungsmethode aufzeigt, um beim Ablauf einer Insufflation den intraabdominellen Druck im Abdomen zu bestimmen.

Diese Insufflationsverfahren und Insufflationssysteme werden in der minimal invasiven Chirurgie und in der interventionellen Laparoskopie eingesetzt, um im Körper einen Untersuchungs- bzw. Operationsraum zu schaffen. Hierzu wird ein Pneumoperitoneum durch die Insufflation von Gas, insbesondere CO₂-Gas erzeugt.

Im Wesentlichen kann man zwei Verfahren der Gasinsufflation unterscheiden.
Dies ist einerseits das Verfahren nach dem "Niederdruckprinzip", bei dem der Insufflationsdruck gleich dem Solldruck bzw. Zieldruck ist, der im Abdomen als abdomineller Druck nicht überschritten werden soll.

Andererseits ist das Verfahren nach dem "Überdruckprinzip" bekannt, bei dem der Insufflationsdruck stufenweise höher als der Solldruck angewendet wird, wobei die Messung des intraabdominellen Druckes zyklisch in Pausen erfolgt, in denen der Insufflationsdruck und Flow auf Null gesetzt wird.

Die Funktion eines Insufflationssystems und des Verfahrens können vereinfachend folgender Maßen beschrieben werden.

Der Insufflator steht über eine Insufflationsleitung mit einem Körper, z.B. auch einem physikalischen Körper, in Fluidverbindung. Um den Körper bis zu einem bestimmten Solldruck bzw. Zieldruck aufzublasen, wird seitens des Insufflators eine Gasströmung, Flow, erzeugt, die mit Überdruck oder beim Solldruck in den Körper geleitet wird. Bei vorhandener Strömung bzw. Flow wird am Insufflator der Insufflationsdruck oder Gerätedruck P_{g} festgestellt. Der zu überwachende Druck im Körper bzw. Abdomen, der einen unkritischen Solldruck P_{S} bzw. Zieldruck P_{Z} nicht überschreiten soll, kann direkt nur in einer Unterbrechungsphase und einem Zurückgehen des Flow auf Null gemessen werden, wobei dann über die Insufflationsleitung der abdominelle Druck P_{abd} als Gerätedruck P_{g} anzeigbar ist.
Der intraabdominelle Druck P_{abd} kann daher nur mit hohem Aufwand direkt gemessen werden und wird daher aus Kosten- und Zeitgründen auf andere Weise rechnerisch oder approximativ erfasst.
Als Information über den Druck im Abdomen stehen daher für die Insufflationsverfahren üblicherweise nur der Druck am Ausgang des Insufflationssystems oder Gerätes P_{g} und der Flow F zur Verfügung.

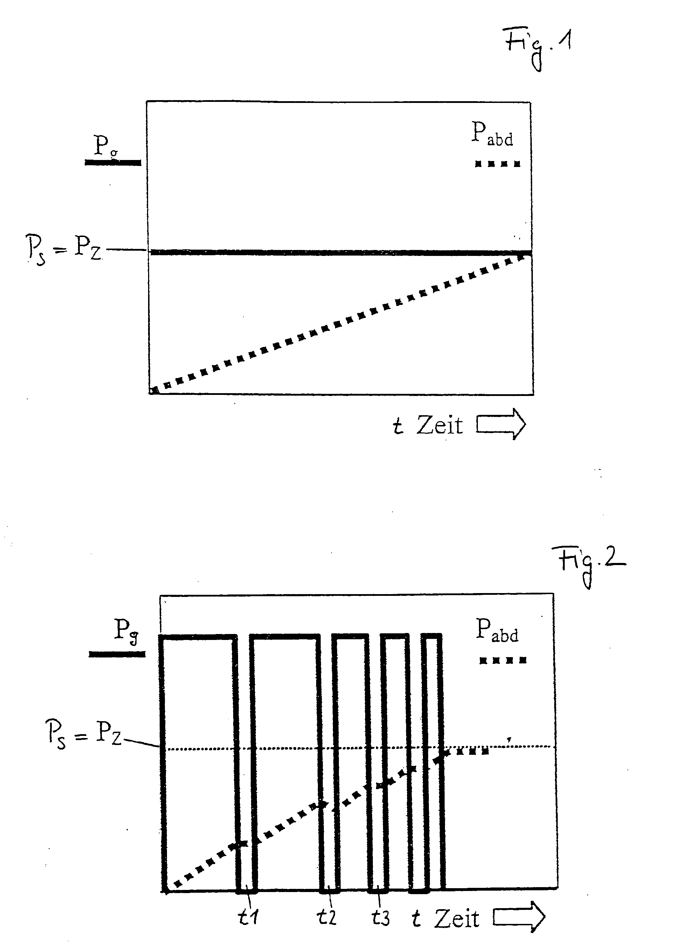

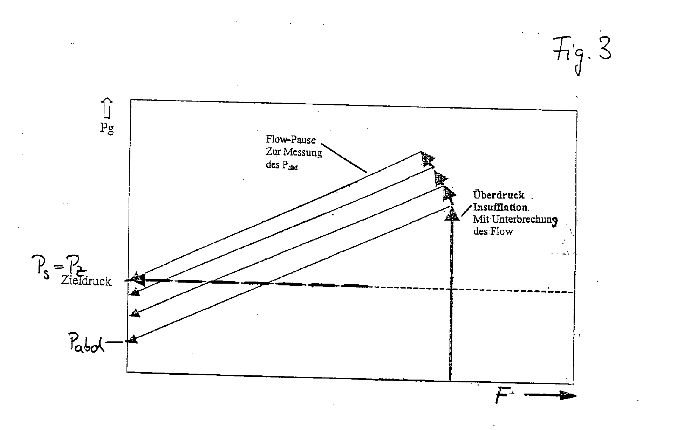

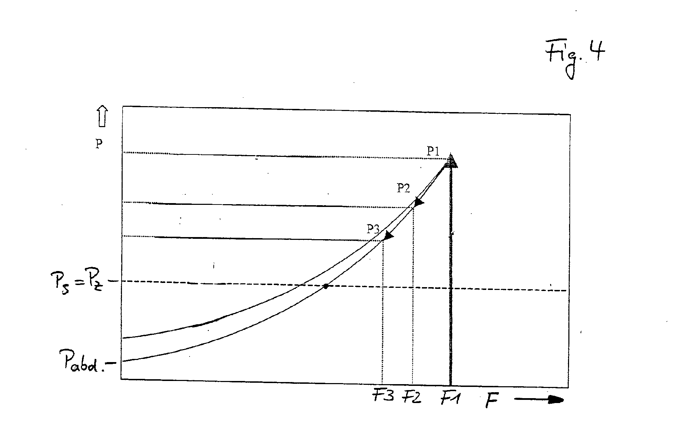

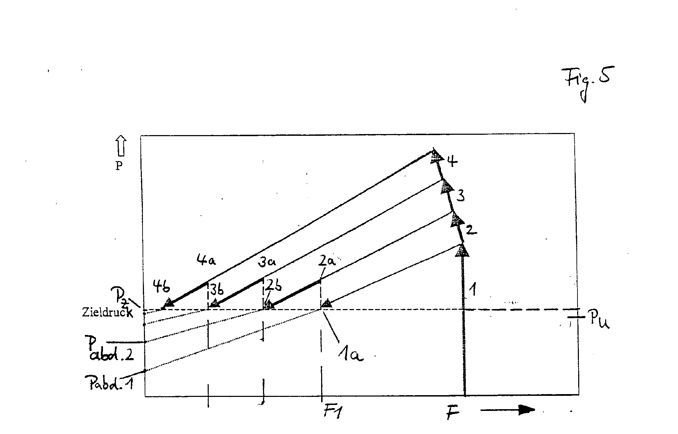

Normalerweise ist bei diesen Verfahren der Druck des Insufflationssystems P_{g} höher als der momentane intraabdominelle Druck P_{abd} und auch deutlich höher als der Zieldruck P_{Z}. Wird kein Flow erzeugt und herrscht Gleichgewicht, so kann der Druck des Insufflationssystems P_{g} gleich dem intraabdominellen Druck P_{abd} gesetzt werden. Diese Verfahrensprinzipien des Niederdruck- und Überdruckverfahrens sind in den Fig. 1 und 2 über die Zeit t dargestellt.

In Fig. 1 ist auf der Ordinate der Insufflationsdruck P_{g} angezeigt, der bei der horizontalen Linie als Solldruck P_{S} bzw. Zieldruck P_{Z} angegeben ist. Die Abszisse stellt die Zeitachse t dar.

Die Punktlinie kennzeichnet hierbei den intraabdominellen Druck P_{abd} . Der Vorteil dieses Niederdruckverfahrens ist, dass der Insufflationsdruck P_{g} maximal auf die Größe des Zieldruckes P_{Z} eingestellt wird und damit kritische Überdruckphasen von vorn herein vermieden werden. Weiterhin ist es vorteilhaft, dass mit einem kontinuierlichen Flow F bei diesem Verfahren gearbeitet werden kann und Druckpeaks während der Insufflation vermieden werden. Zu Beginn des Verfahrens kann es zu Druckpeaks kommen, die jedoch für den intraabdominellen Druck unkritisch sind.

Nachteilig ist jedoch, dass der z.B. im Abdomen gewünschte Zieldruck P_{Z} erst nach relativ langer Zeit erreicht wird und bei plötzlichem Druckverlust im Körper der Gasverlust nicht sofort ausgeglichen werden kann, was in Operationsphasen kritisch bewertet werden muss.

Beim Überdruckverfahren, dessen Druck-Zeit-Verlauf in Fig. 2 dargestellt ist, wird mit einem Insufflationsdruck gearbeitet, der wesentlich über dem Zieldruck P_{Z} liegt. Soll der Zieldruck P_{Z} z.B. bei 14 mmHg liegen, so kann mit einem Überdruck von 50 mmHg gearbeitet werden. Dies ist mit der "durchgezogenen" Linie dargestellt.

Der intraabdominelle Druck P_{abd} ist mit Punkt-Linie gezeigt.

Beim Überdruckverfahren erfolgt daher zunächst eine längere Zeitphase mit hohem Überdruck P_{g}. Zur Messung des intraabdominellen Druckes wird der Überdruck und der Flow in einer Pause t1 auf Null gesetzt und nach einem Druckausgleich über die Insufflationsleitung der intraabdominelle Druck P_{abd} festgestellt. Anschließend erfolgt erneut eine Insufflation mit Überdruck, üblicherweise mit einer kürzeren Zeitdauer.
Dieses Verfahrensprinzip ist daher von hohem Überdruck unterbrochen durch Pausen zu Messzwecken charakterisiert. Auch beim Überdruckverfahren ist daher der Nachteil einer relativ langen Zeitdauer vorhanden, bis der gewünschte Zieldruck P_{Z} als intraabdominelle Druck erreicht wird. Zudem bedarf es stets eines totalen Abschaltens des Flow, damit der intraabdominelle Druck P_{abd} erfasst werden kann.

In Fig. 3 ist dieses Überdruckverfahren im Druck-Flow-Diagramm schematisch dargestellt. Das Diagramm verdeutlicht, dass mit Überdruck und hohem Flow gearbeitet wird, wobei der hohe Flow in einer Pause t1 (Fig. 2) auf Null abgesenkt werden muss, um den intraabdominellen Druck P_{abd} feststellen zu können. Dies wiederholt sich in mehreren Insufflationsphasen, bis der Soll- bzw. Zieldruck P_{Z} erreicht ist.

Der Erfindung liegt daher die **Aufgabe** zugrunde, die vorausgehend angesprochenen Nachteile zu überwinden und ein Verfahren zu konzipieren, das die totalen Flow-Unterbrechungen vermeidet und ohne eine direkte Messung des intraabdominellen Druckes auskommt, jedoch diesen intraabdominellen Druck mit hinreichender Genauigkeit ermitteln kann.

Die Lösung dieser Aufgabe wird durch die Schritte gemäß dem Verfahren nach Anspruch 1 erreicht.

Ein wesentlicher Kerngedanke der Erfindung kann darin gesehen werden, einerseits im Hinblick auf das Erreichen einer kurzen Zeitspanne bis zur optimalen Füllung des Körpers bzw. Abdomens auf den Zieldruck, mit der Insufflation bei Überdruck zu arbeiten. Bei diesen Schritten jedoch nicht bis zu einem Ausgleich des Flow auf Null abzusenken, um den intraabdominellen Druck festzustellen. Vielmehr wird gemäß der Erfindung nach einem Schritt der Überdruckinsufflation der Flow nur bis zum Zieldruck P_{Z} abgesenkt, wobei von diesem Punkt aus relativ genau unter Berücksichtigung empirischerVorgaben undDatenderindieserPhase der Insufflation vorliegende intraabdominelle Druck P_{abd} bestimmt werden kann.
Das erfindungsgemäße Insufflationssystem erkennt daher, wie weit der Zieldruck noch vom intraabdominellen Druck entfernt ist und initiiert einen weiteren Insuffiationsschritt.
Nach dieser weiteren Insufflation bei Überdruck wird erneut der Flow bis zum Zieldruck P_{Z} weitestgehend kontinuierlich abgesenkt. Dies geschieht jedoch auf Grund der Kenntnis vom vorausgehenden Zyklus mit einer sehr raschen Absenkung des Flow auf die Einstellung des Flow F, ventilmäßig vom vorausgehenden Zyklus, so dass im zweiten Schritt nur eine relativ kurse Zeitspanne von der weiteren Absenkung auf den Zieldruck P_{Z} benötigt wird.

Diagrammmäßig lässt sich dies nahezu mit einem sägezahnförmigen Verlauf vergleichen.

Diese Zyklen der Überdruckinsufflation mit quasi-kontinuierlichem Flow und dessen Absenkung auf den Zieldruck P_{Z} sowie dem Vergleich mit dem intraabdominellen Druck, werden so lange fortgesetzt, bis der intraabdominelle Druck P_{abd} weitestgehend dem Zieldruck P_{Z} entspricht oder es wird in der Endphase vom Überdruckverfahren auf Niederdruckverfahren umgestellt.

Um eine Verkürzung der gesamten Insufflationsphase auf den gewünschten Zieldruck P_{Z} bzw. intraabdominellen Druck im Abdomen und Körper zu erreichen, macht sich die Erfindung Überdruck-Flow-Kurven bei der Insufflation von Gasen zu Nutze.
Um den intraabdominellen Druck P_{abd} ohne direkte Messung, aber mit hinreichender Genauigkeit bestimmen zu können, ist es empirisch möglich bei einem bestimmten Insufflations-System und einem bestimmten Füllzustand des Abdomens bzw. Pneumoperitoneums den intraabdominellen Druck in Abhängigkeit vom Flow zu ermitteln.
Der Kurvenverlauf ist in Fig. 4 dargestellt. Messungen unter verschiedensten Verhältnissen haben ergeben, dass die Kurven in guter Näherung durch Parabeln approximiert werden können.
Zur Ermittlung des intraabdominellen Druckes P_{abd} bietet sich nun prinzipiell die Möglichkeit an, den Verlauf der aktuellen, für das gegebene Insufflations-System vorliegenden Druck-Flow-Kurve durch Verminderung des Flows beispielsweise in zwei Stufen, zu ermitteln und dann den intraabdominellen Druck P_{abd} durch Berechnung festzustellen.

Grundsätzlich sind hierfür nur drei Flow- und Druckmessungen notwendig, um diesen Kurvenverlauf eindeutig zu bestimmen.

Voraussetzung für dieses Verfahren ist, dass während der Messung sich der intraabdominelle Druck bedingt durch Strömungswiderstand, Leckagen etc. nicht wesentlich ändert und vor jeder Messung der Druckausgleich erreicht wird, um den Verlauf der Kurve hinreichend genau zu bestimmen.

Bei der Ermittlung der Druck-Flow-Kurven für ein bestimmtes Insufflationssystem ist darauf zu achten, dass keine schnellen Veränderungen der Strömungsverhältnisse vorliegen und der Insufflationsdruck nicht zu hoch wird, da hierdurch Ungenauigkeiten bei der weiteren Extrapolation des Kurvenverlaufes eintreten können.

Es hat sich jedoch gezeigt, dass im Rahmen des erfindungsgemäßen Verfahrens und im Hinblick auf den zeitlichen Aspekt sowie dem Sicherheitsaspekt, den intraabdominellen Druck P_{abd} nicht über den Zieldruck im Abdomen ansteigen zu lassen, diese Aspekte in vorteilhafter Weise erreicht werden.

Beim erfindungsgemäßen Verfahren wird daher zur Vermeidung eines zu hohen intraabdominellen Druckes der Flow bei der Insufflation mit Überdruck nur so weit reduziert, bis der Zieldruck P_{Z} oder ein in der Nähe vorgegebener Solldruck P_{S} erreicht ist. Der dann gemessene Flow F ist ein Maß dafür, wie weit der Istdruck im Abdomen vom Zieldruck P_{Z} entfernt ist, was als Regelabweichung dient und für den weiteren Insufflationsverlauf als Führungsgröße für die Regelung der Flow-Dauer des nächsten Insufflationsschubes verwendet wird.
Hierbei ist die Sicherheit gegeben, dass die Ermittlung des intraabdominellen Druckes P_{abd} im Bereich des Zieldruckes P_{Z} hinreichend genau ist.

Das erfindungsgemäße Verfahren bietet den großen Vorteil, dass weitgehend kontinuierlich insuffliert werden kann, ohne dass der Flow F auf Null heruntergefahren werden muss. Im Rahmen dieses kontinuierlichen Verfahrens der Insufflation und der einzelnen Insufflationsschübe kann die Insufflation in etwa wellenförmig, sinusförmig oder angenähert an einen Sägezahnverlauf durchgeführt werden, wobei der Bezugsdruck im Insufflationssystem bzw. -gerät der Zieldruck P_{Z} ist.

Der Istdruck im Abdomen P_{abd} kann daher als Funktion des Flow F beim Zieldruck P_{Z} bestimmt werden, was zu einer Verkürzung der Insufflationszeit führt, um im Abdomen den gewünschten Druck zu erhalten bzw. auszugleichen und zu erreichen.

Bei der Ermittlung des Istdruckes im Körper bzw. Abdomen wird im Gesamtinsufflationssystem auch der Strömungswiderstand auf Grund der vorhandenen Leitung und der Veress-Nadel mitberücksichtigt.
Zweckmäßigerweise wird das Insufflationsverfahren so durchgeführt, dass abhängig von einer bestimmten Druckdifferenz zwischen Zieldruck P_{Z} und Istdruck im Körper P_{abd} mit Überdruck insuffliert wird oder auf ein Niederdruckverfahren umgeschaltet wird. Diese Insufflationsschübe mit Überdruck werden zyklisch wiederholt und hierbei jeweils der intraabdominelle Druck beim Herunterfahren des Flow auf den Zieldruck P_{Z} im System ermittelt.

Besonders zweckmäßig ist es im Rahmen eines nachfolgenden Insufflationszyklusses mit Überdruck, den Flow sehr rasch vom maximalen Flow auf die Flow-Einstellung beim vorausgehenden Zyklus herunterzufahren und anschließend die weitere Absenkung bis zum Zieldruck P_{Z} zu betreiben. Die langsamere Absenkung des Flow betrifft in diesem Fall nur einen relativ kurzen Zeitbereich, wobei die Größenordnung bei Millisekunden (ms) liegt.

Die quasi-kontinuierliche Absenkung des Flow und die Absenkung des Druckes bis zum Zieldruck P_{Z} vermeidet daher Druckstöße, wie sie in bisherigen Systemen auftreten.

Zur weiteren Verbesserung der Sicherheitsaspekte ist das Verfahren so ausgelegt, dass bei Erreichen eines bestimmten Druckunterschiedes zwischen Zieldruck P_{Z} und intraabdominellen Druck, vom Überdruckverfahren auf das Niederdruckverfahren umgeschaltet wird. Dies kann bei einem vorgebbaren Umschaltdruck erfolgen.
Ist die Druckdifferenz aufgrund von Leckagen, Austausch von Instrumenten jedoch größer, so kann automatisch erneut auf das Überdruckverfahren geschaltet werden.
Zweckmäßigerweise wird der Umschaltdruck auf das Niederdruckverfahren etwa bei 90 % des Zieldruckes vorgegeben.

Die Erfindung wird nachfolgend anhand schematischer Zeichnungen und Diagramme noch näher erläutert. Es zeigen:
- Fig. 1: ein beispielhaftes Diagramm zum bekannten Niederdruckverfahren mit der langsamen Annäherung des intraabdominellen Druckes an den Zieldruck;
- Fig. 2: ein Beispiel eines Diagrammes des Überdruckverfahrens mit Darstellung des Geräte- bzw. Systemdruckes P_{g} über der Zeit, wobei FlowUnterbrechungen zwischen einzelnen Insufflationsschüben vorhanden sind;
- Fig. 3: ein schematisches Diagramm des Überdruckverfahrens mit der Absenkung des Flow auf Null zur Erfassung des intraabdominellen Druckes P_{abd};
- Fig. 4: ein schematisches Druck-Flow-Diagramm zur rechnerischen Ermittlung des intraabdominellen Druckes P_{abd} für ein bestimmtes Insufflationssystem und
- Fig. 5: ein Diagramm zum Druck-Flow-Verlauf gemäß dem erfindungsgemäßen Zieldruck-Flow-Verfahren mit der Absenkung des Flow lediglich bis zum Zieldruck P_{Z} .

In Fig. 4 ist das Druck-Flow-Diagramm dargestellt, das für ein betreffendes Insufflationssystem der erfindungsgemäßen Überlegung zu Grunde liegt.
Die Abszisse zeigt den Flow F, während auf der Ordinate der Druck P als intraabdomineller Druck P_{abd} bzw. Solldruck P_{S} oder Zieldruck P_{Z} aufgetragen ist.

Zur Ermittlung des intraabdominellen Druckes P_{abd} bietet sich prinzipiell die Möglichkeit an, den Verlauf der aktuellen Druck-Flow-Kurve für ein bestimmtes Insufflationssystem bzw. -gerät durch Verminderung des Flow F beispielsweise in zwei oder mehr Stufen zu ermitteln und folglich den intraabdominellen Druck P_{abd} durch weitere Approximation des Druckverlaufes in guter Näherung durch einen Parabelverlauf zu berechnen.
Prinzipiell sind nur drei Flow- und Druckmessungen z.B. P1, P2, P3 erforderlich, um den Kurvenverlauf der Druck-Flow-Kurve eindeutig bestimmen zu können. Die Voraussetzung für diese Berechnungsmethode ist, dass während der Messung an den Punkten P1, P2, P3, der intraabdominelle Druck P_{abd} sich nicht wesentlich ändert und vor jeder Messung der Druckausgleich erreicht wird.
Diese Druck-Flow-Kurven gemäß Fig. 4 berücksichtigen daher für ein bestimmtes Insufflationssystem auch den Leitungswiderstand zwischen Insufflationsgerät und Körper bzw. Abdomen.

Zur empirischen Ermittlung erfolgt daher mit Bezug auf das Diagramm in der Fig. 4 zunächst eine Überdruckinsufflation, wobei die Werte für den Flow F1 und der Druck P1 festgehalten werden. Anschließend erfolgt die Bestimmung der Werte bei F2 und P2 und eine weitere Bestimmung bei F3 und P3.

Auf Grund dieses Kurvenverlaufes zwischen den Kurven P1, P2 und P3 lässt sich mit hinreichender Genauigkeit der intraabdominelle Druck P_{abd} rechnerisch ermitteln.
Die Abweichung der parabelförmigen Kurvenverläufe verdeutlicht den Einfluss von Leitungs-/Strömungswiderstand und Leck in dem entsprechenden Insufflationssystem.

Fig. 5 zeigt schematisch das Zieldruck-Flow-Diagramm für das erfindungsgemäße Verfahren. Im Diagramm ist beispielsweise der gewünschte Zieldruck P_{Z} vorgegeben, der bei der Insufflation in das Abdomen z.B. 14 mmHg nicht überschreiten soll. Dieser Zieldruck P_{Z} wird im Insuffla-tionssystem gemessen und stellt den maximalen Druck dar, den der interabdominelle Druck P_{abd} erreichen soll.

Verfahrensgemäß erfolgt zunächst eine Insufflation 1 mit Überdruck, z.B. 50 mmHg, wobei der Flow dann kontinuierlich abgesenkt wird, bis der Zieldruck P_{Z} erreicht ist. Am Punkt 1a wird einerseits der Flow F1 festgehalten und auf Grund der systemimmanenten Parameter kann unverzüglich der intraabdominelle Druck P_{abd}1 ermittelt bzw. aus entsprechenden Speichern abgerufen werden.

Da für diesen Fall die Druckdifferenz zwischen Zieldruck P_{Z} und intraabdominellen Druck P_{abd} 1 relativ groß ist, wird in einem zweiten Schritt 2 weiterhin mit Überdruck insuffliert. Die Flowabsenkung auf den Punkt 2a erfolgt dann sehr rasch, da die vorausgegangene Ventilstellung des Flows bekannt ist. Erst zwischen Punkt 2a und 2b erfolgt die weitere Absenkung des Flow. Auch für den Punkt 2b wird dann der intraabdominelle Druck P_{abd}2 bestimmt. Die erforderliche Druckdifferenz führt dann systemimmanent dazu, ob mit Überdruck im Schritt 3 weiterverfahren wird. Dies ist im Diagramm nach Fig. 5 der Fall. Es erfolgt erneut ein rasches Absenken des Flow auf den Punkt 3a mit der Ventilstellung entsprechend dem vorausgegangenen Zyklus bei 2b.

In einem nachfolgenden Insufflationsschritt 4 wird bei diesem Verfahren der Punkt 4b erreicht, wobei der intraabdominelle Druck nahezu dem Zieldruck P_{Z} entspricht. In diesem Fall kann von einer weiteren Insufflation abgesehen werden, so fern keine Druckverluste durch Leckagen, Instrumentenwechsel etc. im Abdomen vorliegen.

Im Hinblick auf eine noch höhere Sicherheit des Verfahrens kann beim Erreichen eines Umschaltdruckes P_{U} als intraabdomineller Druck P_{abd} von der Insufflation mit Überdruck auf die Insufflation mit Niederdruck umgeschaltet werden. In diesem Fall wird der verbleibende Druckunterschied bis zum Zieldruck P_{Z} mit weitgehend linear ansteigendem Druckverlauf erreicht. Da diese Druckdifferenz üblicherweise sehr gering ist und z.B. bei einem Zieldruck von P_{Z} von 14 mmHg etwa 1 bis 2 mmHg betragen kann, wird auch für diesen Fall sehr rasch der Zieldruck im Abdomen erreicht.

Das erfindungsgemäße Verfahren gestattet daher bei hoher Zuverlässigkeit und Sicherheit eine wesentlich raschere Insufflation als es die bisherigen Verfahren ermöglichen.

## Patentansprüche

1. Verfahren zur Insufflation von Gas mittels eines Insufflations-Systems in einen Körper, insbesondere in das Abdomen,
bei dem die Insufflation des Gases in Abhängigkeit von einem vorgebbaren Zieldruck im Körper und dem Flow des Gases durchgeführt wird,
bei dem bei einem Istdruck im Körper kleiner als der Zieldruck, das Gas mit Überdruck größer als der Zieldruck insuffliert wird,
bei dem zur Ermittlung der Differenz und Regelung zwischen Zieldruck und Istdruck im Körper, der Flow während der Insufflation mit Überdruck zyklisch bis zum Zieldruck oder einem niedrigeren Solldruck reduziert wird, und
bei dem die gemessene Größe des Flow beim Zieldruck oder Solldruck als Regelgröße zwischen Zieldruck und Istdruck im Körper für die weitere Insufflation verwendet wird.

2. Verfahren nach Anspruch 1,
**dadurch gekennzeichnet,**
**dass** der Istdruck im Körper als Funktion des jeweiligen Flow bei Erreichen des Ziel- oder Solldruckes im System ermittelt wird.

3. Verfahren nach Anspruch 1 oder 2,
**dadurch gekennzeichnet,**
**dass** der Istdruck im Körper in Abhängigkeit vom Strömungswiderstand des Insufflations-Systems ermittelt wird.

4. Verfahren nach einem der Ansprüche 1 bis 3,
**dadurch gekennzeichnet**,
a) dass zur Bestimmung des Istdruckes im Körper der Flow, insbesondere kontinuierlich, bis zum Zieloder Solldruck reduziert wird,
b) dass anschließend bei einer vorgebbaren Druckdifferenz zwischen Zieldruck und Istdruck im Körper mit Überdruck insuffliert wird, und
c) dass diese Verfahrensschritte zyklisch wiederholt werden bis der vorgegebene Zieldruck im Körper erreicht wird.

5. Verfahren nach Anspruch 4,
**dadurch gekennzeichnet, dass** die zyklische Reduzierung des Flow unverzüglich auf die Größe des Flow beim Zieldruck im vorausgegangenen Zyklus erfolgt und
dass anschließend der Flow bis zur Erreichung des Zieldruckes weiter reduziert wird.

6. Verfahren nach einem der Ansprüche 1 bis 5,
**dadurch gekennzeichnet,**
**dass** bei einem Umschaltdruck als vorgebbare Druckdifferenz zwischen Zieldruck und momentanem Istdruck im Körper, die weitere Insufflation im Niederdruckverfahren mit dem Zieldruck als Insufflationsdruck durchgeführt wird.

7. Verfahren nach Anspruch 6,
**dadurch gekennzeichnet,**
**dass** bei einer Druckdifferenz größer als die vorgegebene Druckdifferenz die Insufflation im Überdruckverfahren fortgeführt wird.

8. Verfahren nach Anspruch 6 oder 7,
**dadurch gekennzeichnet,**
**dass** der Umschaltdruck etwa bei 90 % des Zieldruckes gewählt wird.
